# EUROPÄISCHE PATENTSCHRIFT

(11) **EP 1 319 379 B1**
(45) Veröffentlichungstag und Bekanntmachung des Hinweises auf die Patenterteilung: **10.11.2004**
(21) Anmeldenummer: 02027398.3
(22) Anmeldetag: 09.12.2002
(51) Int. Cl.: A61F 5/37

(54) **Orthese zur Behandlung von Verletzungen des A-C-Gelenkes der Schulter**
Orthesis for treatment of injuries to the acromio-clavicular joint of the shoulder
Orthèse pour le traitement des lésions de l'articulation acromio-claviculaire de l'épaule

(30) Priorität: 11.12.2001 DE 20120016 U
(43) Veröffentlichungstag der Anmeldung: 18.06.2003
(73) Patentinhaber: Thämert Orthopädische Hilfsmittel GmbH & Co. KG, 30938 Burgwedel/Grossburgwedel (DE)
(72) Erfinder: Berhorst, Günther, 37287 Wehretal (DE)
(74) Vertreter: Jabbusch, Matthias

(56) Entgegenhaltungen:
- DE-C- 4 314 785
- DE-U- 9 215 341
- US-A- 5 203 763
- US-B1- 6 322 528

## Beschreibung

Die Erfindung betrifft eine Orthese zur Behandlung von Verletzungen des Acromium-Clavicula-Gelenkes (A-C-Gelenk) mit einem Schulterpolster und an dem Schulterpolster angeschlossenen Haltebändern.

Eine ähnliche Vorrichtung ist aus dem deutschen Gebrauchsmuster DE 92 15 341 U bekannt, bei dem zur Stabilisierung der Schulter eines Patienten auf einer Schulter eine Schulterschale angeordnet wird und mit Gurten dort gehalten ist. Aus dem deutschen Gebrauchsmuster DE 201 14 446 U1 ist eine Schultergelenks-Funktionsbandage bekannt, die insbesondere zur Behandlung von Hemiparese-Patienten ausgelegt ist.

Vorrichtungen zur postoperativen Anwendung zur Rückstellung des Oberarms und der Schulter sind aus der DE 41 34 969 A1 und dem deutschen Gebrauchsmuster DE 90 10 801.9 bekannt.

Ein anderes im Bereich der Schulter sehr häufig auftretendes Krankheitsbild in der Chirugie und Orthopädie ist die Zerreißung und Verletzung der Bandstrukturen des Acromium-Clavicula-Gelenkes (A-C-Gelenkes) der Schulter. Die Therapie besteht dabei unter anderem aus der Ruhigstellung des Gelenkes mittels geeigneter Verbände. Es sind bereits Verbände verschiedenster Ausführungen bekannt, die in den meisten Fällen mit einer erheblichen Bewegungseinschränkung der verletzten Schulter und oftmals des ganzen Armes einhergehen. Zudem bieten sie oft dabei nur bedingt eine ausreichende Ruhigstellung. Bei der Zerreißung der Bandstrukturen im A-C-Gelenk kommt es zu einem Hochstand der Clavicula, des Schlüsselbeins, dem sogenannten Klaviertasten-Phänomen. Der angelegte Verband soll nun die Strukturen wieder zusammenfügen und ein Zusammenheilen ermöglichen. Man versucht dies dadurch zu erreichen, indem man Druck auf die Clavicula ausübt, um sie in Richtung Acromium oder Schulter zu drücken. Dies gelingt aber sehr oft nur mit unzureichendem Erfolg. Das heißt, der Patient muß sich nach Wochen der Verbandbehandlung mit unzureichendem Ergebnis unter Umständen einer Operation unterziehen, um die Funktion des Schultergelenkes zu erhalten. Die Ausheilung der Verletzung kann dadurch verzögert werden.

Der Erfindung liegt daher die Aufgabe zugrunde, eine Orthese zu schaffen, die eine ausreichende Stabilität der erkrankten Schulter erreicht und das Gelenk sicher fixiert und dabei eine besonders hohe Bewegungsfreiheit für den Patienten erhält.

Die Lösung dieser Aufgabe erfolgt mit einer Orthese mit den Merkmalen des Patentanspruchs 1. Vorteilhafte Weiterbildungen der Erfindung sind in den Unteransprüchen angegeben.

Erfindungswesentlich ist, daß an dem Schulterpolster mindestens drei Haltebänder angesetzt oder mit diesem verbindbar sind, daß das erste Halteband und das zweite Halteband an einem kurz vor dem Ellenbogengelenk zu positionierenden Unterarmhalter befestigt sind, wobei das erste Halteband vor der Schulter und das zweite Halteband hinter der Schulter anlegbar sind und daß das dritte Halteband zum Verlauf hinter dem Rücken zur gegenüberliegenden Schulter ausgebildet und/oder vorgesehen ist und eine dort zu fixierende Schlaufe aufweist.

Mit einer solchen Orthese sind insbesondere Tossy I/II/III-Verletzungen behandelbar. Diese Orthese eignet sich aber auch bei Verletzungen nach Rockwood Typ III bis Typ VI und auch bei lateralen Schlüsselbein- oder Claviculafrakturen. Dies wird erreicht, indem nicht nur Druck nach unten auf die Clavicula ausgeübt wird, sondern gleichzeitig die Schulter nach oben gezogen wird. Das bedeutet, daß das Gelenk in seine normale Stellung kommt und der Patient dabei seine natürliche Bewegungsfreiheit weitestgehend behält. Die Orthese läßt die natürlichen Bewegungen zu, ohne das Therapieziel zu gefährden. Dies erzeugt bei dem Patienten eine positive Einstellung gegenüber der Verletzung, wodurch der Heilungsprozeß, wie heute allgemein bekannt ist, positiv beeinflußt wird.

Bevorzugt weist das Schulterpolster auf seiner dem Benutzer bzw. der Haut der Benutzers zugewandten Seite eine rutschsichere Auflage, insbesondere eine Silikonauflage, auf. Dadurch wird ein hoher Tragekomfort erreicht und ein Abrutschen des Schulterpolsters verhindert. Das Schulterpolster selbst ist bevorzugt aus einem textilen Material hergestellt. Das textile Material ist dabei zur Schaffung einer ausreichenden Stabilität bevorzugt mit einem Polyurethan getränkt oder kann andere geeignete Verstärkungen, wie beispielsweise aus Aluminium, enthalten. Auch die Gestaltung des Schulterpolsters aus Kunststoff ist in bestimmten Einsatzbereichen vorteilhaft.

Die Haltebänder sind bevorzugt lösbar mit dem Schulterpolster verbunden. Bevorzugt sind dazu im Bereich des Schulterpolsters, eventuell über vergleichsweise kurze Ansatzbänder oder Ansatzstücke, Ringe oder Ösen vorgesehen, durch die oder an denen die Haltebänder längenverstellbar geführt und angesetzt sind und mit einem Klettverschluß lösbar und längenverstellbar fixierbar sind. Die Haltebänder sind dabei bevorzugt an drei voneinander beabstandeten Haftpunkten befestigt. Bevorzugt sind die drei Haft- oder Haltepunkte so an dem Schulterpolster angeordnet, daß die damit verbundenen Haltebänder in einem Winkel von jeweils 120° zueinander von diesen entsprechend angeordneten Haftpunkten ausgerichtet sind. Die Haltebänder sind günsterweise aus einem textilen Material oder aus einem hygienisch unbedenklichen Kunststoffmaterial hergestellt.

Der Unterarmhalter ist bevorzugt als den Unterarm vollständig umschließende Spange oder als Unterarmmanschette ausgebildet. Auch diese ist bevorzugt mit einem doppelten, das heißt an jedem Ende angeordnetem Klettverschluß ausgebildet, so daß ein den Unterarm umschließender Ring gebildet wird. Grundsätzlich könnte der Unterarmhalter aber auch als oben offene Schale ausgebildet sein. Der Unterarmhalter und das Schulterpolster sind bevorzugt aus dem gleichen textilen Material hergestellt. In einer bevorzugten Ausführungsform der Erfindung wird die Orthese derart anlegbar ausgestaltet, daß das erste Band und das zweite Band ausgehend vom Schulterpolster vor und hinter der Schulter verlaufen und sich unter dem Oberarm kreuzen und entsprechend am Unterarmhalter befestigt sind. Dadurch wird eine erhöhte Stabilität und eine gute Fixierung des Schulterpolsters erreicht. Bevorzugt ist dazu das erste Halteband und das zweite Halteband derart an dem Schulterpolster und an dem Unterarmhalter befestigt, daß bei verdrehfreier Anbringung und Ausrichtung der Haltebänder diese sich unterhalb des Oberarms kreuzend ausgerichtet sind. In einer anderen Weiterbildung der Erfindung ist auf der Innenseite des Unterarmhalters eine Rutschsicherung vorgesehen. Diese ist bevorzugt von einer im Unterarmhalter angeordneten Silikoneinlage, bevorzugt mit Noppen oder einer anderen rauhen Struktur, gebildet.

Die am dritten Halteband vorgesehene Schlaufe ist bevorzugt als mit einer Klettverbindung zu öffnende Schlaufe ausgebildet. In einer anderen bevorzugten Ausgestaltung ist die Schlaufe geschlossen und das daran angeordnete Halteband vergleichsweise lang, so daß die Schlaufe einfach über den Arm gezogen werden kann und die Länge des Haltebandes über eine Umlenkung am Kraftpunkt oder Haltepunkt des Schulterpolsters variabel einstellbar und mit Hilfe einer Klettverbindung fixierbar ist.

In einer bevorzugten Weiterbildung der Erfindung ist der Orthese ein erster Haltegurt zugeordnet, der am Handgelenk fixierbar ist und derart ausgelegt und bemessen ist, daß er in Verlängerung des vor dem Körper angewinkelten Unterarms um den Rücken herumgeführt und am Oberarm der verletzten Schulter fixierbar ist. Dies kann entweder mit einer Schlaufe oder mit einer Klettverbindung erfolgen. Dadurch wird der Unterarm in Position gehalten und der Tragekomfort erhöht. In einer anderen bevorzugten Weiterbildung der Erfindung ist der Orthese ein zweiter Haltegurt zugeordnet, der am Handgelenk fixierbar ist und derart ausgelegt und bemessen ist, daß dieser ausgehend von dem vor dem Körper angewinkelten Unterarm nach oben geführt ist und dort im Bereich der der Verletzung gegenüberliegenden Schulter an dem dritten Halteband fixierbar ist. Auch dieser Haltegurt dient der Fixierung des Unterarms vor dem Körper und erhöht den Tragekomfort der Orthese. Die beiden beschriebenen Haltegurte können einzeln, bevorzugt jedoch in Kombination, eingesetzt werden. Bevorzugt weist einer der Haltegurte einen Handgelenkhalter in Form einer Handgelenkmanschette auf, und der andere Gurt ist an diesem Handgelenkhalter fixierbar. Bevorzugt weist der oben beschriebene erste Haltegurt diesen Handgelenkhalter auf, wobei an dem Handgelenkhalter eine Lasche vorgesehen ist, durch die der zweite Haltegurt hindurchführbar ist.

Nachfolgend wird die Erfindung anhand eines in der Zeichnung dargestellten bevorzugten Ausführungsbeispiels weiter erläutert. Im einzelnen zeigen die schematischen Darstellungen in:
- Fig. 1:: eine Vorderansicht eines bevorzugten Ausführungsbeispiels der erfindungsgemäßen Orthese am Patienten;
- Fig. 2:: eine Gesamtansicht eines bevorzugten Ausführungsbeispiels der erfindungsgemäßen Orthese;
- Fig. 3:: eine Ansicht des ersten Haltegurtes der der erfindungsgemäßen Orthese zugeordnet ist;
- Fig. 4:: eine Ansicht des zweiten Haltegurtes, der der erfindungsgemäßen Orthese zugeordnet ist;
- Fig. 5:: eine Vorderansicht eines Ausführungsbeispiels der erfindungsgemäßen Orthese am Patienten einschließlich der Haltegurte;
- Fig. 6:: eine Rückansicht gemäß Fig. 5; und
- Fig. 7:: eine vergrößerte Ansicht des Schulterbereichs mit leicht ausgestrecktem Arm.

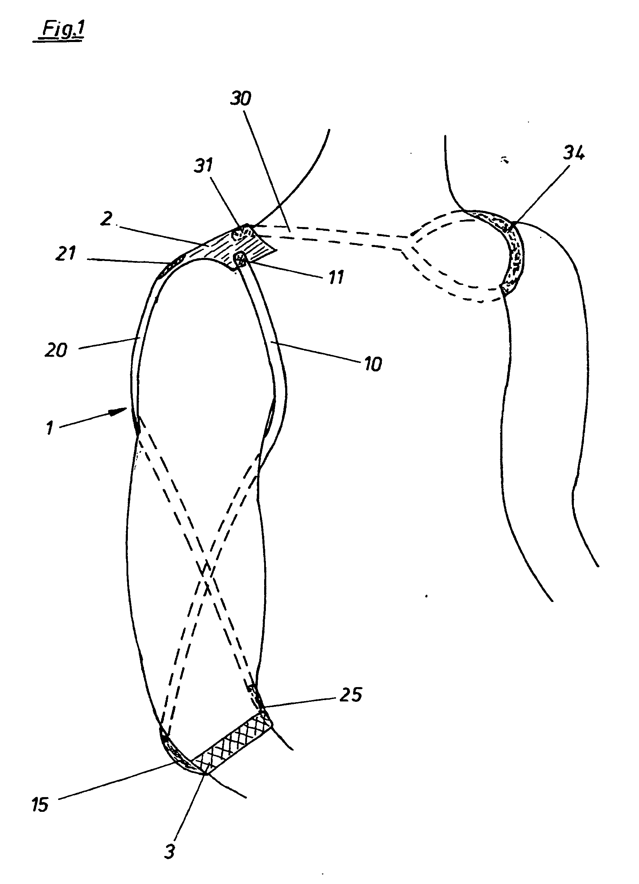

In Fig. 1 ist eine erfindungsgemäße Orthese 1 zur Behandlung von Verletzungen des A-C-Gelenkes der Schulter, insbesondere Tossy I bis III in einer Ansicht von vorne dargestellt. Die Orthese weist im wesentlichen ein Schulterpolster 2, einen Unterarmhalter 3 und drei Haltebänder 10, 20 und 30 auf. Das aus einem Stück gefertigte Schulterpolster 2 ist den anatomischen Gegebenheiten der Schulter angepaßt und weist auf seiner der Haut zugewandten Unterseite ein hautfreundliches Silikonpolster auf. Dieses garantiert einen absolut rutschsicheren Sitz bei einer seitlichen Abduktion des Armes bis zu ca. 85°, bei Ausbildung mit einem Rückengurt sogar noch darüber hinaus. Auf der Oberseite des Schulterpolsters 2 sind drei Haltepunkte 11, 21, 31 angeordnet. Zwei der Haltepunkte 11, 21, nehmen je ein Halteband 10, 20 auf, welche vor und hinter der Schulter in Richtung Ellenbogen, sich auf der Unterseite des Oberarms kreuzend, dort an dem sich am Unterarm befindlichen und an den dort vorhandenen knöcheren Strukturen Halt findenden, spangenförmig den Arm umschließenden Unterarmhalters 3 mittels Haftpunkten 15 und 25 befestigt ist. Der dritte Haltepunkt 31 auf dem Schulterpolster 2 dient zur Befestigung eines quer über den Rücken zur gegenüberliegenden Schulter verlaufenden Haltebandes 30, der dort in eine weich gepolsterte Schlaufe 34 ausläuft. Auch dieses Halteband 30 ist als Klettband ausgeführt. Das Schulterpolster 2 sowie der Unterarmhalter 3 können sowohl in Kunststoff, als auch in textilem Material hergestellt werden. Bei einer Ausführung in textilem Material empfiehlt sich das Einbringen von verstärkenden Elementen, wie zum Beispiel Formteilen aus leicht formbaren Werkstoffen, insbesondere solche aus Aluminium. Das erste Halteband 10 und das zweite Halteband 20 haben bevorzugt eine Breite von ca. 30mm bis 40mm und sind aus sogenanntem Kofferband oder ähnlichem Material gefertigt, welches sich durch seine flexible Beschaffenheit den anatomischen Formen und Gegebenheiten bestens anpaßt. Das dritte Halteband 30 wird aus einem weich gepolsterten Material ausgeführt. Soweit die Haltebänder 10, 20 und 30 und die Schlaufe 34 gestrichelt dargestellt sind, verlaufen diese hinter dem Arm bzw. dem Rücken und sind bei Betrachtung in der dargestellten Perspektive nicht sichtbar.

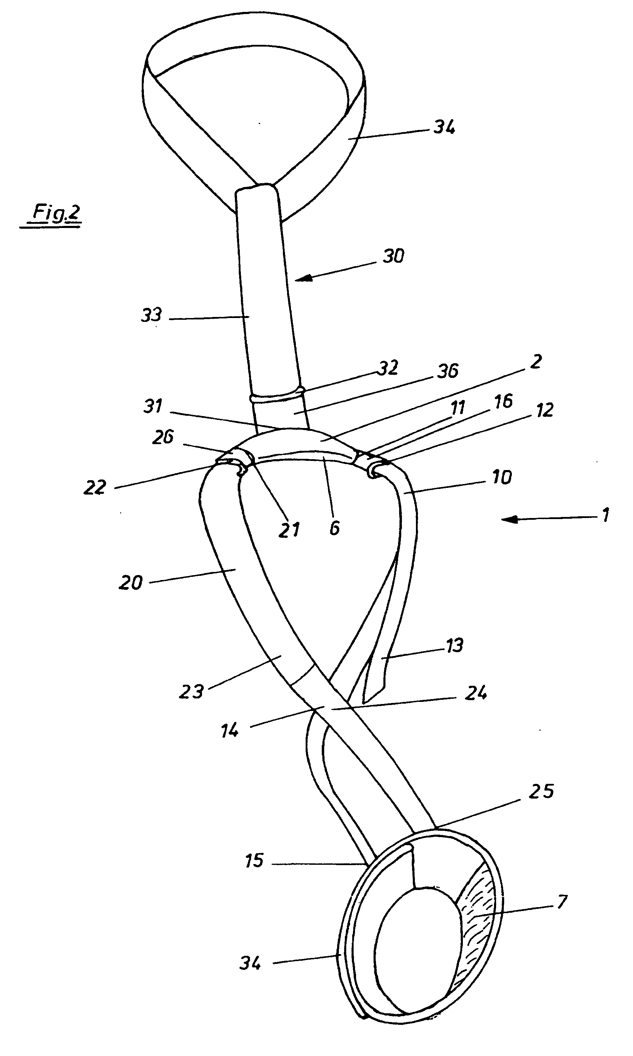

In Fig. 2 ist die erfindungsgemäße Orthese 1 mit dem Schulterpolster 2, den Haltebändern 10, 20 und 30 und dem Unterarmhalter 4 dargestellt. Das Schulterpolster 2 hat eine im wesentlichen ovale Grundform, wobei jedoch auf der der Schulter zugewandten Seite eine kreissegment- oder sichelförmige Ausnehmung 6 vorgesehen ist. Zu beiden Seiten dieser sichelförmigen Ausnehmung 6 sind das erste Halteband 10 und das zweite Halteband 20 angesetzt. An den Haft- oder Haltepunkten 11 und 21 sind kurze, etwa einige Zentimeter lange Ansatzstücke 16 und 26 befestigt, insbesondere angenäht, und an diesen sind Verbindungsringe 12 und 22 befestigt, durch die das erste Halteband 10 bzw. das zweite Halteband 20 längenverstellbar hindurchgeführt sind. In gleicher Weise ist auf der der sichelförmigen Ausnehmung 6 gegenüberliegenden Seite am Haltepunkte 31 ein Ansatzstück 36 angenäht, an dem ebenfalls ein Umlenkring 32 befestigt ist, an dem das Halteband 30 längenverstellbar befestigt ist. Die drei Haltepunkte 11, 21 und 31 sind an beabstandet voneinander angeordneten Positionen des Schulterpolsters 2 angeordnet und die entsprechend daran angeordneten Haltebänder schneiden sich bei gedachter Verlängerung jeweils unter einem Winkel von etwa 120°. Die Haltebänder 10 und 20 sind an Haltepunkten 15 und 25 derart an dem Unterarmhalter 3 angeschlossen bzw. dort befestigt, daß bei einer verdrehfreien Anlage der Haltebänder 10 und 20 diese sich im Bereich der Punkte 14 und 24 kreuzen. Dieser Kreuzungspunkt liegt unterhalb des Oberarms und führt im Ergebnis zu einer zusätzlichen Stabilisierung des Schulterpolsters 2. Dies insbesondere deshalb, da der Unterarmhalter 3, der bevorzugt als geschlossene Spange oder hier textilartige Manschette ausgebildet ist, auf seiner Innenseite eine Rutschsicherung 7 aufweist, die von einem Silikonpolster gebildet ist. Der Abstand zwischen dem Schulterpolster 2 und dem Unterarmhalter 3 läßt sich mit Hilfe der Haltebänder 10 und 20 einstellen. Die Haltebänder 10 und 20 sind durch die Umlenkringe 12 und 22 geführt und können nach gewünschter Längeneinstellung mit ihren an den Enden befindlichen Klettbereichen 13 und 23 an den Gurten 10 und 20 fixiert werden. Der dritte Haltegurt 30 weist in seinem Endbereich eine Schlaufe 34 auf, die vom Schulterpolster 2 aus hinter dem Rücken verlaufend zur gegenüberliegenden Schulter gelegt wird. Zur Längeneinstellung wird das dritte Halteband 30 durch den Umlenkring 32 geführt, der über ein Ansatzstück 36 am Haltepunkt 31 befestigt ist und mit Hilfe des am Ende des dritten Haltebandes vorgesehenen Klettbereichs 33 auf diesem befestigt.

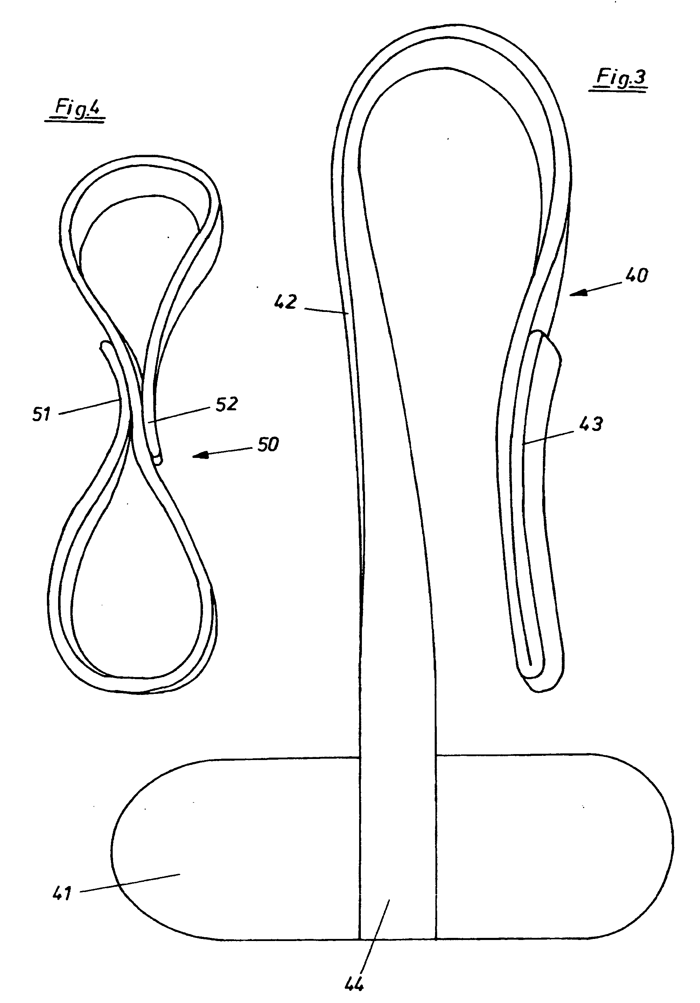

In Fig. 3 ist eine Ansicht eines ersten Haltegurtes 40 dargestellt. Dieser wird mit einem Handgelenkhalter 41, der als ringförmig schließende Handgelenkmanschette ausgebildet ist und mit einem Klettverschluß verschlossen werden kann, am Handgelenk fixiert. In einem zentralen Bereich des Handgelenkhalters 41 ist ein Band 42 angeordnet, das in Verlängerung des vor dem Körper angewinkelten Unterarms um den Rücken herumgeführt wird und um den Oberarm an der verletzten Schulter geführt ist und dort mit Hilfe des Klettbereichs 43 im Endbereich des Bandes 42 fixiert wird. Auf dem Handgelenkhalter 41, also auf der Außenseite des Handgelenkhalters 41, ist in Fortsetzung oder linearer Erstreckung des Bandes 42 eine offene Lasche 44 auf dem Handgelenkhalter 41 angeordnet.

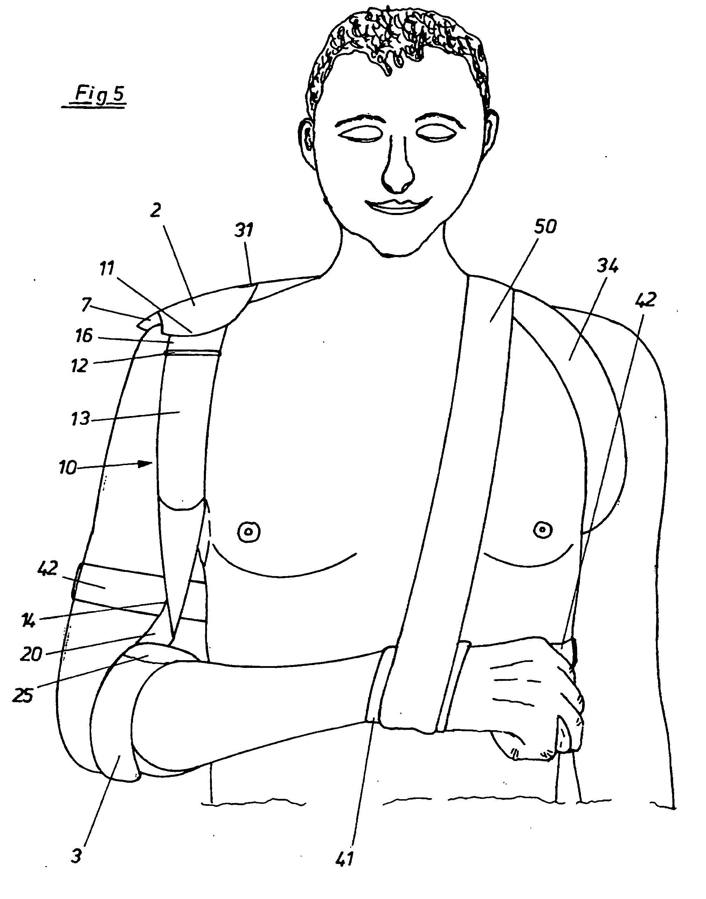

In Fig. 4 ist ein zweiter Haltegurt 50 dargestellt, der ebenfalls zur Fixierung des vor dem Körper angewinkelten Unterarms vorgesehen ist. Ein Endbereich 51 des zweiten Haltegurtes 50 wird um den vertikal ausgerichteten Unterarm bzw. das Handgelenk geführt und kann dabei durch die dann ebenfalls vertikal offene, horizontal ausgerichtete Lasche 44 geführt werden, so daß ein seitliches Verrutschen verhindert ist. Mit Hilfe eines im Endbereich 51 vorgesehenen Klettbereichs kann in diesem Bereich eine Schlaufe gebildet werden. Der andere Endbereich 52 des zweiten Haltegurtes 50 weist ebenfalls einen Klettbereich auf, der im Bereich der der zu behandelnden Schulter gegenüberliegenden Schulter an dem dort verlaufenden dritten Halteband 30 der Orthese 1 fixierbar ist.

In Fig. 5 ist eine Vorderansicht eines bevorzugten Ausführungsbeispiels einer erfindungsgemäßen Orthese 1 mit den der Orthese zugeordneten ersten 40 und zweiten Haltegurten 50 am Patienten dargestellt. Im wesentlichen ist das Schulterpolster 2, der Unterarmhalter 3 und das am Haltepunkt 11 ansetzende Halteband 10 dargestellt. Ausgehend vom Haltepunkt 11 ist zunächst ein Ansatzstück 16 und daran anschließend ein Umlenkring 12 an dem Schulterpolster 2 angeordnet. Das Halteband 10 ist durch den Umlenkring 12 geführt und mit Hilfe seines im Endbereich angeordneten Klettbereichs 14 längenjustiert fixiert. Unterhalb des Oberarms kreuzt sich im Bereich des Kreuzungspunktes 14 das erste Halteband 10 mit dem zweiten Halteband 20, das im Verbindungspunkt 25 an dem Unterarmhalter 3 befestigt ist. Weiterhin ist am Schulterpolster 2 oberhalb der Außenseite der Schulter der sichelförmig abgeschnittene Bereich 7 erkennbar. Auf der gegenüberliegenden Seite ist der Haltepunkt 13 für das dritte Halteband vorgesehen, das in der um die gegenüberliegende Schulter gelegten Schlaufe 34 endet. Zur Fixierung des Unterarms vor dem Körper ist dieser mit den Haltegurten 40 und 50 fixiert. Der Haltegurt 40 weist eine Handgelenkmanschette 41 auf, von der ausgehend ein Band 42 um den Rücken herumgelegt ist und auf der linken Seite der Zeichnung um den gegenüberliegenden Oberarm geführt ist und dort mit Hilfe eines Klettverschlusses fixiert ist. Der zweite Haltegurt 50 verbindet das Handgelenk mit der der verletzten Schulter gegenüberliegenden Schulter und hält den Arm auf diese Weise hoch.

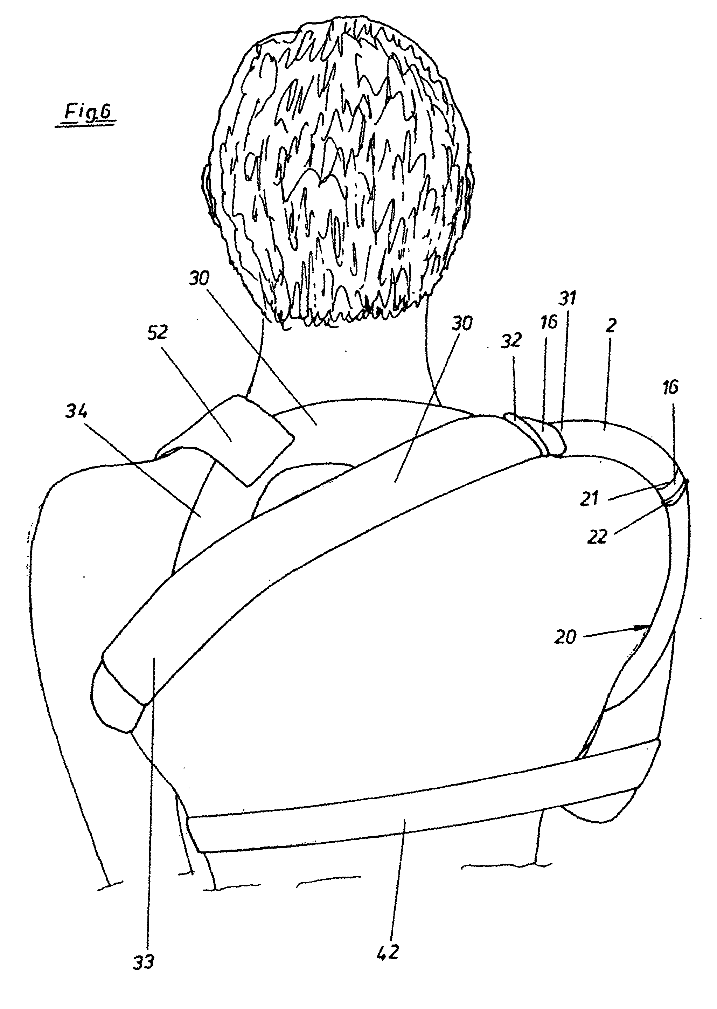

In Fig. 6 ist die Rückenansicht der angelegten erfindungsgemäßen Orthese gemäß Fig. 5 dargestellt. Ausgehend von dem Schulterpolster 2 an hier sichtbaren Haltepunkten 21 und 31 und den daran angeordneten Ansatzstücken 26 und 36 mit den Umlenkringen 22 und 32 geht, ausgehend vom Umlenkring 22, das zweite Halteband 20 hinter dem Rücken zu dem aus dieser Perspektive nicht sichtbaren Unterarmhalter. Das dritte Halteband 30 mit der in seinem Endbereich angeordneten Schlaufe 34, die um die gegenüberliegende Schulter gelegt ist, wird durch den Umlenkring 32 geführt und in seiner Länge angepaßt. Der Endbereich des dritten Haltebandes 30 kann mit seinem dort angeordnetem Klettbereich 33 je nach Länge entweder auf dem Gurt 30 oder, so wie hier dargestellt, auf der Schlaufe 34 fixiert werden. In diesem Schulterbereich ist auch das Ende 52 des zweiten Haltegurtes 50 auf dem dritten Haltegurt 30 bzw. der Schlaufe 34 mit Hilfe einer Klettverbindung fixiert. Weiterhin ist hier das Band 42 des ersten Haltegurtes 40 zu sehen, das um den Rücken herumgelegt wird und am Oberarm in der Figur rechts fixiert wird.

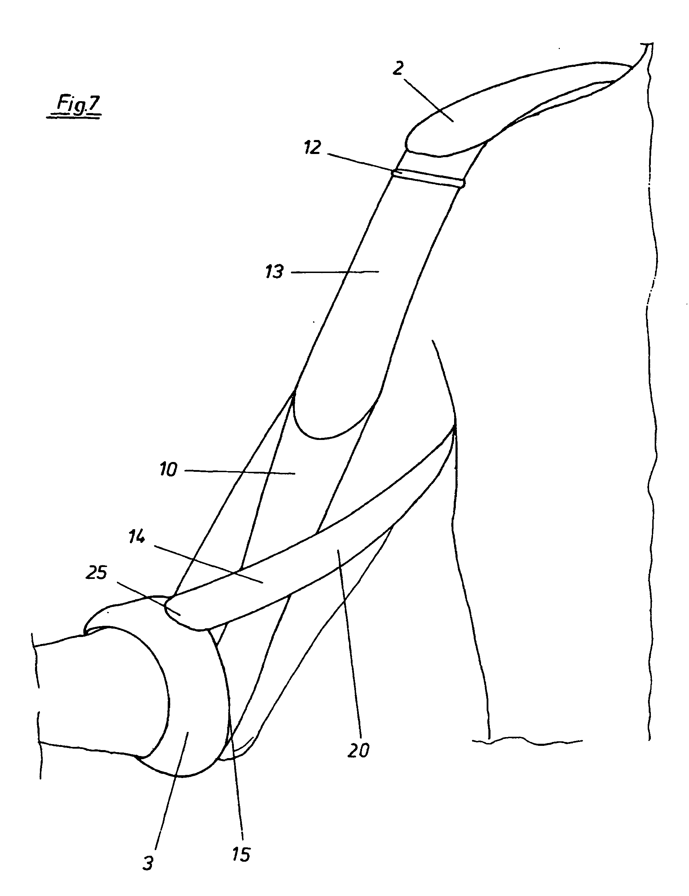

In Fig. 7 ist eine vergrößerte Ansicht im Bereich des Oberarms dargestellt, in der insbesondere die sich kreuzenden Haltebänder dargestellt sind. Das erste Halteband 10 und das zweite Halteband 20 sind in Verbindungspunkten 15 und 25 derart am Unterarmhalter 3 befestigt, insbesondere angenäht, daß bei entsprechender Befestigung des ersten Haltebandes 10 und des zweiten Haltebandes 20 am Schulterpolster 2 bei verdrehfreier Ausrichtung des ersten Haltebandes 10 und des zweiten Haltebandes 20 sich diese in einem Kreuzungspunkt 14 kreuzen. Aufgrund der verrutschsicheren Fixierung des Unterarmhalters 3 führt diese voneinander beabstandete Anbringung oder Fixierung der Haltebänder 10 und 20 in den Punkten 15 und 25 an dem Unterarmhalter 3 zu einem besonders sicheren Sitz der Orthese.

## Patentansprüche

1. Orthese zur Behandlung von Verletzungen des Acromium-Clavicula-Gelenkes mit einem Schulterpolster (2) und an dem Schulterpolster angeschlossenen Haltebändern (10, 20, 30),
wobei an dem Schulterpolster (2) mindestens drei Haltebänder (10, 20, 30) angesetzt oder mit diesem verbindbar sind, **dadurch gekennzeichnet,**
**daß**
das erste Halteband (10) und das zweite Halteband (20) an einem Kurz vor dem Ellenbogengelenk zu positionierendem Unterarmhalter (3) befestigt sind, wobei das erste Halteband (10) vor der Schulter und das zweite Halteband (20) hinter der Schulter anlegbar ist,
**daß** das dritte Halteband (30) zum Verlauf hinter dem Rücken zur gegenüberliegenden Schulter vorgesehen ist und eine dort zu fixierende Schlaufe (34) aufweist.

2. Orthese nach Anspruch 1, **dadurch gekennzeichnet, daß** das Schulterpolster (2) auf seiner dem Benutzer bzw. der Haut des Benutzers zugewandten Seite eine rutschsichere Auflage, insbesondere eine Silikonauflage, aufweist.

3. Orthese nach einem der Ansprüche 1 oder 2, **dadurch gekennzeichnet, daß** das Schulterpolster (2) aus einem textilen Material besteht.

4. Orthese nach einem der vorhergehenden Ansprüche, **dadurch gekennzeichnet, daß** die Haltebänder (10, 20, 30) lösbar mit dem Schulterpolster (2) verbunden sind.

5. Orthese nach einem der vorhergehenden Ansprüche, **dadurch gekennzeichnet, daß** die drei Haltebänder (10, 20, 30) an drei voneinander beabstandeten Haltepunkten (11, 21, 31) des Schulterpolsters (2) befestigt sind.

6. Orthese nach einem der vorhergehenden Ansprüche, **dadurch gekennzeichnet, daß** der Unterarmhalter (3) als Unterarmspange ausgebildet ist.

7. Orthese nach einem der vorhergehenden Ansprüche, **dadurch gekennzeichnet, daß** das erste Halteband (10) und das zweite Halteband (20) derart an dem Schulterpolster (2) und an dem Unterarmhalter (3) befestigt sind, daß bei verdrehfreier Anbringung der Haltebänder (10, 20) diese sich unterhalb des Oberarms kreuzend ausgerichtet sind.

8. Orthese nach einem der vorhergehenden Ansprüche, **dadurch gekennzeichnet, daß** auf der Innenseite des Unterarmhalters (3) eine Rutschsicherung (7) vorgesehen ist.

9. Orthese nach einem der vorhergehenden Ansprüche, **dadurch gekennzeichnet, daß** die am dritten Halteband (30) vorgesehene Schlaufe (34) als mit einer Klettverbindung zu öffnende Schlaufe ausgebildet ist.

10. Orthese nach einem der vorhergehenden Ansprüche, **dadurch gekennzeichnet, daß** der Orthese ein erster Haltegurt (40) zugeordnet ist, der am Handgelenk fixierbar ist und derart ausgelegt und bemessen ist, daß er in Verlängerung des vor dem Körper angewinkelten Unterarms um den Rücken herumgeführt und am Oberarm der verletzten Schulter fixierbar ist.

11. Orthese nach einem der vorhergehenden Ansprüche, **dadurch gekennzeichnet, daß** der Orthese ein zweiter Haltegurt (50) zugeordnet ist, der am Handgelenk fixierbar ist und derart ausgelegt und bemessen ist, daß dieser ausgehend von dem vor dem Körper angewinkelten Unterarm nach oben geführt ist und dort im Bereich der der Verletzung gegenüberliegenden Schulter an dem dritten Halteband fixierbar ist.

12. Orthese nach Anspruch 11 im Kombination mit Anspruch 10, **dadurch gekennzeichnet, daß** einer der Haltegurte einen Handgelenkhalter (41) aufweist und der andere Gurt an diesem Handgelenkhalter (41) fixierbar ist.

## Claims

1. An orthesis for treatment of injuries to the acromio-clavicular joint of the shoulder, including a shoulder pad (2) and retaining straps (10, 20, 30) attached to the shoulder pad (2), wherein at least three retaining straps (10, 20, 30) are attached to the shoulder pad (2) or may be tied thereto,
**characterised in that**
the first retaining strap (10) and the second retaining strap (20) are secured to a lower arm fastener (3) to be positioned slightly below the elbow joint, wherein the first retaining strap (10) can be applied in front of the shoulder and the second retaining strap (20) can be applied behind the shoulder,
that the third retaining strap (30) is designed to extend behind the back to the opposite shoulder and is furnished with a loop (34) for securing it there.

2. The orthesis according to claim 1,
**characterised in that**
the side of the shoulder pad (2) facing the user or the user's skin is furnished with a non-slip surface layer, particularly a silicone layer.

3. The orthesis according to either of claims 1 or 2, **characterised in that**
the shoulder pad (2) is made from a textile material.

4. The orthesis according to any of the preceding claims, **characterised in that**
the retaining straps (10, 20, 30) are detachably connected to the shoulder padding (2).

5. The orthesis according to any of the preceding claims, **characterised in that**
the three retaining straps (10, 20, 30) are secured at a distance from each other at three affixing points on the shoulder pad (2).

6. The orthesis according to any of the preceding claims, **characterised in that**
the lower arm fastener (3) is configured as a lower arm clasp.

7. The orthesis according to any of the preceding claims, **characterised in that**
the first retaining strap (10) and the second retaining strap (20) are secured to the should pad (2) and the lower arm fastener (3) in such manner that when the retaining straps (10, 20) are applied without twisting, they are aligned to cross one another below the upper arm.

8. The orthesis according to any of the preceding claims, **characterised in that**
an anti-slip element (7) is provided on the inside of the lower arm fastener (3).

9. The orthesis according to any of the preceding claims, **characterised in that**
the loop (34) that is provided on the third retaining strap (30) is constructed as a loop that may be opened by a hook and loop fabric fastening.

10. The orthesis according to any of the preceding claims, **characterised in that**
a first retaining belt (40) is assigned to the orthesis and is attachable at the wrist and is of such a construction and size that it extends beyond the lower arm bent in front of the body and can be passed round the back and affixed to the upper arm of the injured shoulder.

11. The orthesis according to any of the preceding claims, **characterised in that**
a second retaining belt (50) is assigned to the orthesis and is attachable at the wrist and is of such a construction and size that, starting from the lower arm bent in front of the body it passes upwards and can be affixed to the third retaining strap on the uninjured shoulder.

12. The orthesis according to claim 11 in conjunction with claim 10,
**characterised in that**
one of the retaining belts has a wrist support (41) and the other belt can be affixed to this wrist support (41).

## Revendications

1. Orthèse pour le traitement de blessures de l'articulation acromio-claviculaire, comprenant une épaulette (2) et des bandes de maintien (10, 20, 30) reliées à celle-ci, au moins trois bandes de maintien (10, 20, 30) étant posées sur l'épaulette (2) ou aptes à être reliées à celle-ci,
**caractérisée en ce que** la première bande de maintien (10) et la deuxième bande de maintien (20) sont fixées à un support d'avant-bras (3) à placer juste avant l'articulation du coude, la première (10) étant apte à être mise devant l'épaule, et la deuxième (20) derrière l'épaule,
et **en ce que** la troisième bande de maintien (30) est destinée à s'étendre derrière le dos, jusqu'à l'épaule opposée, et présente une boucle (34) à fixer à cet endroit.

2. Orthèse selon la revendication 1, **caractérisée en ce que** l'épaulette (2) présente sur son côté tourné vers l'utilisateur ou vers la peau de l'utilisateur une couche antidérapante, en particulier une couche de silicone.

3. Orthèse selon la revendication 1 ou 2, **caractérisée en ce que** l'épaulette (2) se compose d'une matière textile.

4. Orthèse selon l'une des revendications précédentes, **caractérisée en ce que** les bandes de maintien (10, 20, 30) sont reliées de manière amovible à l'épaulette (2).

5. Orthèse selon l'une des revendications précédentes, **caractérisée en ce que** les trois bandes de maintien (10, 20, 30) sont fixées à trois points de fixation (11, 21, 31) de l'épaulette (2) qui sont espacés les uns des autres.

6. Orthèse selon l'une des revendications précédentes, **caractérisée en ce que** le support d'avant-bras (3) est conçu comme une boucle pour avant-bras.

7. Orthèse selon l'une des revendications précédentes, **caractérisée en ce que** la première bande de maintien (10) et la deuxième bande de maintien (20) sont fixées à l'épaulette (2) et au support d'avant-bras (3) de telle sorte que si elles sont montées pour pouvoir tourner librement, elles se croisent sous le bras.

8. Orthèse selon l'une des revendications précédentes, **caractérisée en ce qu'**un dispositif antidérapant (7) est prévu sur le côté intérieur du support d'avant-bras (3).

9. Orthèse selon l'une des revendications précédentes, **caractérisé en ce que** la boucle (34) prévue sur la troisième bande de maintien (30) est conçue comme une boucle à ouvrir à l'aide d'une liaison auto-agrippante.

10. Orthèse selon l'une des revendications précédentes, **caractérisée en ce qu'**on associe à l'orthèse une première sangle de maintien (40) qui est apte à être fixée au poignet et qui est conçue et dimensionnée pour passer autour du dos, dans le prolongement de l'avant-bras plié devant le corps, et pour pouvoir être fixée au bras de l'épaule blessée.

11. Orthèse selon l'une des revendications précédentes, **caractérisée en ce qu'**on associe à l'orthèse une seconde sangle de maintien (50) qui est apte à être fixée au poignet et qui est conçue et dimensionnée pour s'étendre vers le haut à partir de l'avant-bras plié devant le corps, et pour pouvoir être fixée à la troisième bande de maintien dans la zone de l'épaule opposée à la blessure.

12. Orthèse selon la revendication 11 combinée à la revendication 10, **caractérisée en ce que** l'une des sangles de maintien comporte un support de poignet (41) tandis que l'autre sangle est apte à être fixée audit support (41).
